# FASCICULE DE BREVET EUROPEEN

(11) **EP 0 861 095 B1**
(45) Date de publication et mention de la délivrance du brevet: **02.05.2002**
(21) Numéro de dépôt: 96931115.8
(22) Date de dépôt: 12.09.1996
(51) Int. Cl.: A61L 9/01, A61L 9/04

(54) **GRANULES DESODORISANTS POUR CENDRIERS**
DESODORIERENDE GRANULATE FÜR ASCHENBECHER
DEODORANT GRANULATES FOR ASHTRAYS

(30) Priorité: 13.09.1995 FR 9510987
(43) Date de publication de la demande: 02.09.1998
(73) Titulaire: Jean, Marcel, 06250 Mougins (FR)
(72) Inventeur: Jean, Marcel, 06250 Mougins (FR)
(74) Mandataire: Roman, Michel
(86) Numéro de dépôt international: FR9601405
(87) Numéro de publication internationale: WO9710009

(56) Documents cités:
- EP-A- 0 250 304
- GB-A- 2 024 014
- GB-A- 2 217 603

## Description

La présente invention a pour objet des granulés désodorisants pour cendriers.

Ils sont destinés à garnir le fonds des cendriers de tous types recevant des cendres de tabac. Ils peuvent en effet être utilisés aussi bien pour les cendriers de table, que ce soit chez des particuliers ou dans des salles de bars ou de restaurants, que pour les cendriers à grande contenance disposés dans les lieux publics tels que halls de gares, aérodromes ou grandes surfaces commerciales, ou encore pour des cendriers de voitures.

Il est notoire que les odeurs de tabac, et particulièrement celles qui émanent des cendriers sont parmi les plus indésirables, une des odeurs les plus désagréables étant celle du tabac froid.

Le développement rapide ces dernières années des campagnes anti-tabac a suscité une législation de plus en plus restrictive avec en particulier l'expansion des zones non fumeurs dans les restaurants et les lieux publics.

Les solutions trouvées jusqu'ici pour supprimer la perception des odeurs de tabac émanant des cendriers ont une portée fort limitée et, pour obtenir un résultat appréciable, on a dû les associer à des techniques de masquage afin de couvrir l'odeur du tabac qualifiée de désagréable, par une autre odeur perçue comme agréable.

Il s'en suit le plus souvent que les performances désodorisantes sont inégales, les parfums utilisés étant généralement perçus comme trop violents, ce qui affecte le plaisir de celui qui fume, sans pour autant vraiment satisfaire le non fumeur qui bien souvent a imposé l'utilisation du produit "anti-tabac" au fumeur. Ceci est particulièrement vrai dans le cas des désodorisants anti-tabac de cendrier.

Il est évident qu'un produit qui saurait supprimer les odeurs du tabac émanant du cendrier, dont la diffusion serait accélérée dès l'instant où l'on commence à fumer, et qui serait capable de remplir l'atmosphère d'une senteur agréable à tous, dans l'environnement immédiat, restaurant, bureau, salons, etc, pourrait faire retrouver une convivialité "naturelle" entre fumeurs et non fumeurs.

Aujourd'hui les produits anti-tabac existants pour cendrier ont pour support:
- soit des sables fins non poreux, colorés et parfumés par enrobage, ou poreux mais avec peu de capacité d'accueil de produit actif du fait de leur finesse.
- soit des granulés de plus grosse taille en matériaux divers, par exemple en matière plastique.

Leur produit actif privilégie soit la performance désodorisante, soit la couverture de l'odeur par des parfums très violents, mais aucun produit n'associe efficacement les 3 éléments nécessaires à un bon résultat final:
- Un système désodorisant réduisant significativement la perception désagréable de l'odeur du tabac et en particulier celle du tabac froid.
- un parfum de grande qualité suffisamment volatil pour être diffusé quasi instantanément dès qu'il est touché par l'extrémité incandescente de la cigarette.
- un support incombustible capable d'accueillir, de retenir et relarguer les produits actifs au bon moment, c'est-à-dire dès qu'il entre en contact avec l'extrémité incandescente de la cigarette.

Le produit selon la présente invention a pour objectif de remédier à cet état de choses. Il permet en effet, à un prix très raisonnable, de neutraliser intégralement les odeurs de tabac du cendrier, tout en parfumant délicatement l'ambiance et ceci sans dénaturer le plaisir du fumeur, apportant ainsi le maximum de confort olfactif.

Le désodorisant pour cendriers est formé d'un composé actif comprenant un parfum de qualité corporelle ou aromatique, un solvant peu volatil et un déodorant à base d'acide undécylénique, ledit composé étant intégré dans des granulés constitués d'un support minéral incombustible d'une granulosité déterminée pour ne pas laisser de trace de poussière à l'utilisation, et d'une porosité permettant d'accueillir une quantité importante de produit actif, et de le relarguer au contact de la cigarette allumée.

La description détaillée ci-après se rapporte à un exemple non limitatif d'une des formes de réalisation de l'objet de l'invention.

Le produit objet de l'invention se présente sous la forme de granulés associant un support à point de fusion élevée et à granulométrie bien définie, un parfum de haute qualité, un solvant de viscosité élevée à faible évaporation, et un principe désodorisant.

Le support est un élément déterminant pour l'efficacité de la facilité d'utilisation du produit. Il doit avoir le point de fusion le plus élevé possible, la pierre ponce ou pierre de lave qui a un point de fusion de 1500 °C est parfaite à cet égard.

Sa granulométrie doit être parfaitement définie: trop fin, il produira une poussière non souhaitée par l'utilisateur, ou n'accueillera pas correctement des proportions importantes d'actif; trop gros il remplira mal sa fonction. Une bonne taille des granulés est de 2 à 3 mm dans leur plus petite dimension, et 5 à 8 mm dans la plus grande. On utilisera avantageusement des granulés légèrement polis en galets.

Ce produit est courant dans l'industrie où il y a de nombreuses applications comme abrasif, son prix de revient est très bas. Enfin, sa porosité (densité 0.6 environ) permet d'accueillir les 15 à 20 % d'actif nécessaire pour avoir une efficacité optimum en garnissant simplement d'une mince couche le fond du cendrier. Le temps d'imprégnation est très court, en quelques minutes les granulés sont parfaitement secs, ainsi la senteur ne se transferera pas dans la cigarette posée dans le cendrier, même non allumée.

Le parfum utilisé est du type utilisé pour la parfumerie corporelle, conforme aux prescriptions reconnues par les professionnels de la parfumerie, et en particulier à la norme française de l'IFRA et aux recommandations du bureau américain RIFM.

Ce type de parfum doit être suffisamment volatil pour que le confort olfactif du parfumage d'ambiance soit en tout point comparable à celui du parfumage corporel. Il contiendra avantageusement des hespéridés (citron, en particulier) qui ont depuis toujours démontré leur fonction désodorisante.

Il pourra tout aussi agréablement restituer des senteurs alimentaires ou gourmandes présentant le même confort olfactif.

Le solvant sera de préférence du monopropylène glycol, mais pourra consister également en polypropylène glycol ou un composé ayant des caractéristiques similaires.

Le propylène glycol est largement reconnu comme un des meilleurs supports de parfum de qualité, capable par sa viscosité d'agglomérer les éventuelles traces de poussière, et par sa faible évaporation de réguler la sortie du parfum incorporé dans un support poreux. Il s'agit d'un solvant présentant un point de fusion assez élevé conférant une inflammabilité réduite aux matières aromatiques en abaissant suffisamment le point éclair du produit actif pour provoquer l'auto extinction des petites flammes qui pourraient se former dans le cas, par exemple de présence d'allumettes enflammées dans le cendrier.

Le principe désodorisant retenu est l'acide undécylénique (connu sous l'appellation de "C11"), obtenu à partir de l'huile de ricin, et qui a démontré son efficacité lorsque le dosage est parfaitement maîtrisé.

Il est utilisé dans le cas présent sous la forme d'undécylénate d'éthyle qui est un ester de l'acide undécylénique et de l'éthanol dont la qualité cosmétique est reconnue. Un dosage approprié permet d'utiliser son odeur propre comme un des éléments constitutifs du parfum, ce que notre expérimentation a permis de démontrer.

Pour former le produit actif, parfum et solvant seront avantageusement associés dans des proportions comprises entre 20/80 et 50/50, et pour le principe désodorisant, les résultats les plus probants ont été obtenus en incorporant sensiblement 3% d'undécylénate d'éthyle,

Ce produit actif rentrera ensuite pour 15 à 20% en poids dans le support minéral retenu pour cette application.

On peut colorer sans difficulté les granulés en incorporant des colorants agréés en parfumeries directement dans le produit actif, à raison d'environ 3% de solution contenant 2 à 4% de colorant dans de l'eau ou du monopropylène glycol.

Dans un but publicitaire ou commercial, on pourra visualiser la fonction désodorisante en colorant différemment une petite partie des granulés. Ainsi, à titre d'exemple, une petite quantité de grains verts dans un produit de couleur différente pourra symboliser la présence d'un désodorisant d'origine naturelle.

Les granulés pour cendrier ainsi réalisés par assemblage d'un produit actif constitué de parfum, de monopropylène glycol, d'undécylénate d'éthyle et de colorant incorporé dans des granulés de pierre ponce, apportent un maximum d'efficacité et des résultats inconnus à ce jour, appréciés aussi bien par le fumeur que par le non fumeur.

Placés dans le cendrier des automobiles de non fumeurs, ils entretiendront une ambiance fraîche et discrète pendant de longs mois, la diffusion pouvant être ajustée en ouvrant plus ou moins le cendrier.

Le fumeur dispose d'un produit qui ne dénature pas son plaisir. En effet, la nature volatile des parfums spécialement formulés fait qu'ils sont évaporés dès le début du contact avec l'extrémité incandescente de la cigarette ou du cigare. Il n'y a donc pas de parfumage intempestif de l'arôme du tabac, son plaisir est intact.

Un dosage approprié de l'acide undécylénique rend l'effet désodorisant quasiment total, ce qui peut être constaté aussi bien en sentant la fumée évaporée depuis le cendrier qui ne présente aucune odeur désagréable de tabac, qu'en sentant le cendrier vidé après utilisation, où l'on ne sent pratiquement aucune odeur résiduelle, ni ensuite l'odeur si désagréable de tabac froid.

L'effet parfumant est immédiat, et intervient au bon moment. Les granulés au repos dans le cendrier agissent relativement faiblement, mais dès le contact avec l'extrémité incandescente de la cigarette, les granulés voient leur parfum rapidement évaporé et entraîné par la fumée dans l'atmosphère avec l'acide undécylénique. Il n'y a plus de naissance de mauvaises odeurs de tabac à partir du cendrier.

L'effet parfumant étant amené à son niveau optimum par la qualité du parfum qui s'évapore, l'atmosphère n'est virtuellement plus polluée par la fumée rejetée par le fumeur. Ceci est le premier souhait du non fumeur. On peut donc aller jusqu'à prétendre que l'effet obtenu est de nature à réhabiliter la cohabitation des fumeurs et non fumeurs.

Le positionnement des divers éléments constitutifs donne à l'objet de l'invention un maximum d'effets utiles qui n'avaient pas été, à ce jour, obtenus par des produits similaires.

## Revendications

1. Granulés désodorisants pour cendriers, destinés à garnir le fonds des cendriers de tous types revevant des cendres de tabac, qu'il s'agisse de cendriers de table utilisés chez des particuliers ou dans des salles de bars ou de restaurants, ou de cendriers à grande contenance disposés dans les lieux publics tels que halls de gares, aérodromes ou grandes surfaces commerciales, ou encore de cendriers de véhicules,
**caractérisé par** la combinaison d'un composé actif comprenant un parfum de qualité corporelle ou aromatique, un solvant de viscosité élevée à faible évaporation et un déodorant à base d'undécylénate d'éthyle, ledit composé étant intégré dans des granulés minéraux incombustibles dont la granulosité est déterminée pour ne pas laisser de trace de poussière à l'utilisation, et présentant une porosité permettant d'absorber au moins 15% en poids de composé actif, et de le relarguer au contact de la cigarette ou du cigare allumés.

2. granulés désodorisants selon la revendication 1, se caractérisant par le fait que le solvant est du monopropylène glycol.

3. granulés désodorisants selon l'une quelconque des revendications précédentes, se caractérisant par le fait que le matériau constitutif du support est de la pierre ponce ou pierre de lave d'une densité égale à 0.6 environ.

4. granulés désodorisants selon l'une quelconque des revendications précédentes, se caractérisant par le fait que le support est constitué de granulés légèrement polis en galets de 2 à 3 mm dans leur plus petite dimension, et 5 à 8 mm dans la plus grande.

5. granulés désodorisants selon l'une quelconque des revendications précédentes, se caractérisant par le fait que le parfum utilisé est du type utilisé pour la parfumerie corporelle et ne contient que des matières premières conformes aux prescriptions des organismes professionnels de la parfumerie française (IFRA) et américaine (RIFM).

6. granulés désodorisants selon l'une quelconque des revendications 1 à 4, se caractérisant par le fait que le parfum utilisé est du type aromatique à odeurs alimentaires.

7. granulés désodorisants selon l'une quelconque des revendications précédentes, se caractérisant par le fait que le parfum et le solvant sont associés dans des proportions comprises entre 20 parties de parfum pour 80 parties de solvant, et 50 parties de parfum pour 50 parties de solvant.

8. granulés désodorisants selon l'une quelconque des revendications précédentes, se caractérisant par le fait que le composé actif comporte sensiblement 3% d'undécylénate d'éthyle.

9. granulés désodorisants selon l'une quelconque des revendications précédentes, se caractérisant par le fait qu'il comportent 15 à 20% de composé actif.

10. granulés désodorisants selon l'une quelconque des revendications précédentes, se caractérisant par le fait qu'ils sont colorés par incorporation dans le composé actif de colorants agréés en parfumeries, à raison d'environ 3% de solution contenant 2 à 4% de colorant dans de l'eau ou du monopropylène glycol.

## Patentansprüche

1. Desodorierendes Granulat für Aschenbecher, um deren Boden zu bedecken und die Tabakasche aufzunehmen, sowohl für Tischaschenbecher in Privathaushalten oder in Gaststätten, wie für großvolumige, an öffentlichen Orten, zum Beispiel auf Bahnhöfen, an Flughäfen oder in Einkaufszentren, aufgestellte Aschenbecher ebenso wie für Aschenbecher in Fahrzeugen,
**gekennzeichnet durch** die Kombination einer aktiven Zusammensetzung, in der ein Parfüm in Kosmetik-Qualität oder ein Aromastoff, ein Lösungsmittel mit hoher Viskosität und geringer Verdunstung sowie ein Desodorant auf Basis eines Ethyl-Undecylenats enthalten ist, wobei die betreffende Zusammensetzung in das unbrennbare mineralische Granulat eingebracht wird, dessen Korngröße so festgelegt ist, dass keine Staubspuren beim Gebrauch hinterlassen werden und dessen Porosität ausreicht, um mindestens 15 Gew.-% der aktiven Zusammensetzungen zu absorbieren und es im Kontakt mit einer brennenden Zigarette oder Zigarre wieder freizusetzen.

2. Desodorierendes Granulat nach Anspruch 1, **gekennzeichnet dadurch, dass** das Lösungsmittel Monopropylen Glycol ist.

3. Desodorierendes Granulat gemäß einem der vorstehenden Ansprüche, **gekennzeichnet dadurch, dass** das Trägermaterial Bimsstein oder Lava mit einer Dichte von etwa 0,6 ist.

4. Desodorierendes Granulat nach einem der vorstehenden Ansprüche, **gekennzeichnet dadurch, dass** die Körner des Granulats leicht poliert sind, ihre kleinsten Abmessungen 2 bis 3 mm und ihre größten 5 bis 8 mm betragen.

5. Desodorierendes Granulat nach einem der vorstehenden Ansprüche, **gekennzeichnet dadurch, dass** das verwendete Parfüm der gleichen Art ist, wie es für die Kosmetik verwendet wird und nur Rohstoffe enthält, die den Vorschriften der Berufsorganisationen der französischen (IFRA) und amerikanischen (RIFM) Parfümindustrie entsprechen.

6. Desodorierendes Granulat nach einem der Ansprüche 1 bis 4, **gekennzeichnet dadurch, dass** das verwendete Parfüm ein aromatischer Typ mit dem Geruch von Nahrungsmitteln ist.

7. Desodorierendes Granulat nach einem der vorstehenden Ansprüche, **gekennzeichnet dadurch, dass** das Parfüm und das Lösungsmittel in Proportionen zugesetzt werden, die zwischen 20 Teilen Parfüm auf 80 Teile Lösungsmittel und 50 Teilen Parfüm auf 50 Teilen Lösungsmittel liegen.

8. Desodorierendes Granulat nach einem der vorstehenden Ansprüche, **gekennzeichnet dadurch, dass** die aktive Zusammensetzung in etwa 3 % Ethyl-Undecylenat enthält.

9. Desodorierendes Granulat nach einem der vorstehenden Ansprüche, **gekennzeichnet dadurch, dass** es 15 bis 20 % der aktiven Zusammensetzung enthält.

10. Desodorierendes Granulat nach einem der vorstehenden Ansprüche, **gekennzeichnet dadurch, dass** es durch für kosmetische Produkte zugelassene Farbstoffe gefärbt wird, die der aktiven Zusammensetzung in einem Anteil von etwa 3 % einer Lösung mit 2 bis 4 % Farbstoff in Wasser oder in Monopropylen Glycol zugesetzt werden.

## Claims

1. Deodorizing granulates for use in ashtrays, intended to cover the bottom of ashtrays of all types receiving tobacco ash, whether table ashtrays used in private homes or in bars or restaurants, or large capacity ashtrays in public places such as station or airport halls or large shopping areas, or vehicle ashtrays,
**characterized by** the combination of an active compound comprising a body or aromatic quality perfume, a solvent of high viscosity with low evaporation and an ethyl decylenate based deodorant, the aforementioned compound being incorporated in fireproof mineral pellets whose particle size is determined so as not to leave traces of dust when used, and having sufficient porosity to absorb at least 15% by weight of active compound, and to salt it out on contact with the lit cigarette or cigar.

2. Deodorizing granulates according to claim 1, **characterized by** the fact that the solvent is mono-propylene glycol.

3. Deodorizing granulates according to any of the preceding claims, **characterized by** the fact that the material constituting the support is pumice or lava stone with a density of approximately 0.6.

4. Deodorizing granulates according to any of the preceding claims, **characterized by** the fact that the support consists of slightly polished granulates in 2 to 3 mm pebbles at their smallest, and 5 to 8 mm at their largest.

5. Deodorizing granulates according to any of the preceding claims,
**characterized by** the fact that the perfume used is of the type used for body perfumery and contains only raw materials in conformity with the regulations of the French and American professional perfumery organizations (IFRA and RIFM respectively).

6. Deodorizing granulates according to any of claims 1 to 4, **characterized by** the fact that the perfume used is of the aromatic food odor type.

7. Deodorizing granulates according to any of the preceding claims, **characterized by** the fact that the perfume and the solvent are associated in proportions ranging between 20 parts of perfume to 80 parts of solvent, and 50 parts of perfume to 50 parts of solvent.

8. Deodorizing granulates according to any of the preceding claims, **characterized by** the fact that the active compound comprises approximately 3% of ethyl decylenate.

9. Deodorizing granulates according to any of the preceding claims, **characterized by** the fact that it includes 15 to 20% of active compound.

10. Deodorizing granulates according to any of the preceding claims, **characterized by** the fact that they are colored by incorporation of perfume industry approved colorants in the active compound at a rate of approximately 3% of solution containing 2 to 4% of colorant in water or mono-propylene glycol.
